# EUROPÄISCHE PATENTSCHRIFT

(11) **EP 0 649 831 B1**
(45) Veröffentlichungstag und Bekanntmachung des Hinweises auf die Patenterteilung: **14.10.1998**
(21) Anmeldenummer: 94116175.4
(22) Anmeldetag: 13.10.1994
(51) Int. Cl.: C07C 69/65, C07C 67/343

(54) **Verfahren zur Herstellung von 3-(p-Fluorphenyl)-2-methylpropionsäure und 3-(p-Fluorphenyl)-2-methylpropionsäurederivaten**
Process for the preparation of 3-(p-fluorphenyle)-2-methylepropionic acid and of derivatives of 3-(p-fluorphenyle)-2-methylepropionic acid
Procédé de préparation de l'acide 3-(p-fluorophényle)-2-méthylepropionique et de dérivés de l'acide 3-(p-fluorophényle)-2-méthylepropionique

(30) Priorität: 20.10.1993 DE 4335748
(43) Veröffentlichungstag der Anmeldung: 26.04.1995
(73) Patentinhaber: HOECHST AKTIENGESELLSCHAFT, 65929 Frankfurt am Main (DE)
(72) Erfinder: Beller, Matthias, Dr., D-65527 Niedernhausen (DE); Fischer, Hartmut, Dr., D-65719 Hofheim (DE); Strutz, Heinz, Dr., D-61250 Usingen (DE)

(56) Entgegenhaltungen:
- EP-A- 0 508 586
- EP-A- 0 553 668

## Beschreibung

Die vorliegende Erfindung betrifft ein neues Verfahren zur Herstellung von 3-(p-Fluorphenyl)-2-methyl-propionsäure und 3-(p-Fluorphenyl)-2-methyl-propionsäurederivaten.

3-(p-Fluorphenyl)-2-methyl-propionsäure und 3-(p-Fluorphenyl)-2-methyl-propionsäurederivate haben technische Bedeutung als Pharmazwischenprodukte. Insbesondere sind sie als Zwischenprodukte für Analgetica, Antipyretica und Antiphlogistica wie Sulindac von Bedeutung.

Bisherige Synthesen derartiger Zwischenprodukte gehen beispielsweise von dem entsprechenden p-Fluorbenzaldehyd aus, der in Gegenwart stöchiometrischer Mengen Base mit Propionsäureanhydrid umgesetzt wird. Reduktion mit Wasserstoff in Gegenwart von Palladium auf Aktivkohle als Katalysator liefert dann die β-Aryl-2-methyl-propionsäure (US 3,654,349).

β-Aryl-2-methyl-propionsäure kann auch durch Reaktion des p-Fluorbenzaldehyds mit einem substituierten Essigsäureester in einer Claisen-Reaktion oder mit einem halogenierten Propionsäureester in einer Reformatsky-Reaktion hergestellt werden (DE 2 039 426).

Die beschriebenen Verfahren weisen folgende Nachteile auf:

Der eingesetzte p-Fluorbenzaldehyd ist eine technisch nur sehr teuer (> 80 DM/kg) herzustellende Verbindung. Außerdem fallen bei der Herstellung des Ausgangsmaterials größere Mengen an Salzen an, was die Gesamtsynthese ökologisch zweifelhaft macht.

Bei der nachfolgenden Umsetzung des p-Fluorbenzaldehyds mit Propionsäureanhydrid oder mit einem halogenierten Propionsäureester fallen erneut große Mengen an Salzen an. In den genannten Patentschriften werden keine Angaben bezüglich der Ausbeute der Reaktion von p-Fluorbenzaldehyd mit Propionsäureanhydrid gemacht. Ausgehend von analogen Beispielen wie der Reaktion von 2,4-Difluorbenzaldehyd ist anzunehmen, daß die Ausbeute nur 70 bis 80 % beträgt.

Die EP-A-0 553 668 beschreibt ein Verfahren zur Herstellung von Methacrylsäurederivaten unter Verwendung einer Carbonsäure als Lösungsmittel. Die mit diesem Verfahren erzielte Ausbeute an Methacrylsäurederivaten beträgt nur bis zu 49 %.

Aus der EP-A-0 508 586 ist ein Verfahren zur Hydrierung von speziellen Aryl-methacrlysäurederivaten zu 3-(p-Fluorphenyl)-2-methyl-propionsäurederivaten in Gegenwart eines Palladiumkatalysators bekannt. Die Herstellung der Arylmethacrlysäurederivaten aus einem Diazoniumsalz über ein Methacrylsäurederivat ist nicht beschrieben.

Es bestand somit ein erhebliches Bedürfnis nach einem Verfahren, das 3-(p-Fluorphenyl)-2-methyl-propionsäure und ihre Derivate in möglichst hoher Ausbeute ohne störende Verunreinigungen und in einfacher Weise liefert und das zusätzlich eine einfache technische Umsetzung erlaubt.

Diese Aufgabe wird gelöst durch ein Verfahren zur Herstellung von 3-(p-Fluorphenyl)-2-methyl-propionsäure und deren Derivaten der Formel (I), 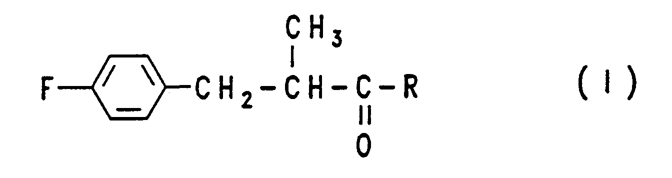 in welcher R eine Hydroxygruppe, eine lineare oder verzweigte (C₁-C₈)-Alkoxygruppe, eine Aryloxygruppe, eine Aminogruppe oder eine (C₁-C₈)-Alkylaminogruppe oder eine (C₁-C₈)-Dialkylaminogruppe darstellt, dadurch gekennzeichnet, daß man
a) p-Fluoranilin in das p-Fluorphenyldiazoniumsalz der Formel (II) überführt 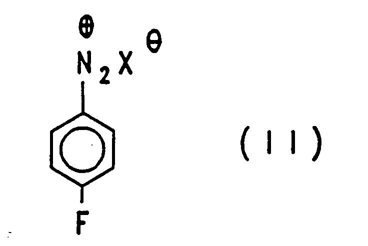 in welcher X das Äquivalent eines Anions einer organischen oder anorganischen Säure mit einem pKa-Wert kleiner 7 darstellt
b) das Diazoniumsalz der Formel (II) mit einem Methacrylsäurederivat der Formel (III) 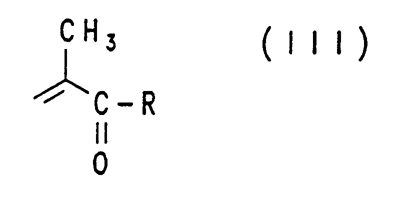 in welcher R die oben angegebene Bedeutung besitzt, in Gegenwart eines Palladiumkatalysators gegebenenfalls in Gegenwart einer Base und/oder eines Lösungsmittels zu den Verbindungen der Formeln (IV) und (V), in welchen R die oben angegebene Bedeutung besitzt, umsetzt 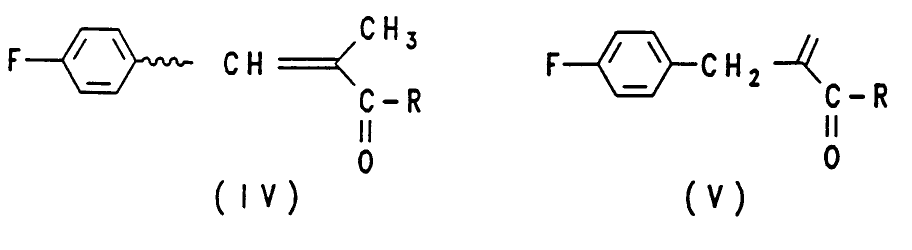
c) die erhaltenen Verbindungen der Formeln (IV) und (V) in Gegenwart eines Palladiumkatalysators mit Wasserstoff hydriert.

In einer bevorzugten Reaktionsvariante werden die Diazotierung und die Olefinierung des Diazoniumsalzes in einem Schritt durchgeführt, so daß die Herstellung des gewünschten Produktes in nur zwei Schritten erzielt werden kann.

Hinsichtlich der Bedeutung des Restes R kann der Substituent R beispielsweise eine Methoxy-, Ethoxy-, n-Propyloxy, Isopropyloxy-, n-Butyloxy-, i-Butyloxy-, tert.-Butyloxy-, n-Pentyloxy-, i-Pentyloxy-, n-Hexyloxy-, n-Haptyloxy-, n-Octyloxy-, 2-Ethylhexyloxy-, 2-Ethyldecyloxy-, n-Decyloxy-, n-Dodecyloxy-, Hydroxy-, ferner eine N,N-Dimethylamino-, N,N-Diethylamino-, N-Methylamino-, N-Ethylamino-, N,N-Methylethylamino-, N,N-Dipropylamino-, N,N-Dibutylamino-, N-Piperidyl-, N-Morpholino-, N-Acetamido-, N-Propionsäureamid-, bevorzugt eine Hydroxy-, Methoxy-, Ethoxy-, n-Propyloxy-, Isopropyloxy-, Butoxy-, N,N-Dimethylamino-, N,N-Diethylamino-, besonders bevorzugt eine Hydroxy-, Methoxy-, Ethoxy-, darstellen.

In Stufe b) kann der Palladiumkatalysator prinzipiell homogen als auch heterogen eingesetzt werden. Geeignete homogene Palladiumkatalysatoren sind beispielsweise Palladium(II)acetat, Palladium(II)chlorid, Lithiumtetrachloropalladat und Palladium(II)bromid. Ein besonderer Vorteil besteht in der Verwendung heterogener Palladiumträgerkatalysatoren. Dabei wird der Palladiumkatalysator zweckmäßigerweise auf einem Trägermaterial wie beispielsweise Aktivkohle, Calciumcarbonat, Bariumsulfat, Bimsstein, Tonerde, Kieselgur, Kieselgel, Graphit, Magnesiumoxid und/oder Aluminiumoxid angewendet. Eine andere Möglichkeit ist die Verwendung von Palladiumbisbenzylidenacetonkomplexen, die in einigen Lösungsmitteln, beispielsweise Methanol, heterogen vorliegen. Palladiumbenzylidenacetonkomplexe, sind Palladiumbisbenzylidenaceton, substituierte Palladiumbisbenzylidenacetonkomplexe wie 3,3'-4,4'-Tetramethoxy-bisbenzylidenaceton-Palladium-Komplexe, Dimethoxybisbenzylidenaceton-Palladium-Difluorbisbenzylidenaceton-Palladium-Komplexe. Bevorzugt wird Palladium auf Aktivkohle, Palladium(II)acetat oder Palladiumbisbenzylidenacetonkomplex zur Anwendung gebracht.

Hinsichtlich des Mengenverhältnisses Palladium zu Diazoniumsalz hat es sich bewährt, etwa 0.001 bis etwa 20 mol. %, vorzugsweise 0,1 bis etwa 1,0 mol. % Palladium, bezogen auf das Diazoniumsalz anzuwenden.

Was das Mengenverhältnis der Reaktanden anbelangt, so ist es zweckmäßig 1 mol Diazoniumsalz der allgemeinen Formel (III) mit etwa 0,8 - 20 mol, insbesondere mit 1 - 5 mol, Methacrylsäurederivat zur Umsetzung zu bringen.

Das Verfahren der Stufe b) wird vorzugsweise in protischen Lösungsmitteln wie Wasser, Methanol, Ethanol oder wäßrigen Lösungen der Alkohole durchgeführt. Es kann bei bestimmten Diazoniumsalzen von Vorteil sein, das Verfahren in situ, d.h. ohne Isolierung der Diazoniumsalze in einer wäßrigen Lösung durchzuführen.

Besonders vorteilhaft an dem erfindungsgemäßen Verfahren ist, daß Katalysatormengen unter 1 mol% für die Olefinierung des Diazoniumsalzes ausreichen. Außerdem kann auf Basenzusatz verzichtet werden.

Die entstehenden Produkte der Stufe b) liegen in der Regel als Gemisch von 3-(p-Fluorphenyl)methacrylsäurederivaten und 2-(p-Fluorbenzyl)-acrylsäurederivaten vor. Das Isomerenverhältnis der beiden Produkte beträgt dabei in der Regel zwischen 4:1 bis 1:1. Es ist jedoch möglich, unter besonderen Bedingungen das Isomerenverhältnis zu verändern. Da die Doppelbindung der entstehenden 3-(p-Fluorphenyl)methacrylsäurederivate und 2-(p-Fluorbenzyl)-acrylsäurederivate im Rahmen der weiteren Synthese hydriert wird, ist das Isomerenverhältnis unkritisch.

Die Hydrierung mit Palladiumkatalysatoren führt in hohen Ausbeuten zu den 3-(p-Fluorphenyl)-2-methyl-propionsäurederivaten.
Das neue Verfahren ist aufgrund der katalytischen Reaktionsführung und des billigeren Ausgangsmaterials (Preis 4-Fluoranilin 22 DM/kg; Preis 4-Fluorbenzaldehyd 80 DM/kg) alten Verfahren ökonomisch und ökologisch deutlich überlegen. Außerdem beträgt die Gesamtausbeute des Verfahrens 80 bis 85 %, was den bisher beschriebenen Verfahren überlegen ist.

Die nachstehenden Beispiele dienen zur Erläuterung des erfindungsgemäßen Verfahrens, ohne es darauf zu beschränken.

### Beispiel 1:

71,5 g 4-Fluoranilin werden in 246.6 g Tetrafluorborsäure (50 %ig) gelöst und 10 Minuten bei 0 bis 5°C gerührt. Dann addiert man 47,9 g Natriumnitrit in 55 ml Wasser innerhalb von 20 Minuten bei 0 bis 5°C zu. Das ausgefallene Produkt wird mit kaltem Ether gewaschen.
Ausbeute: 96 % 4-Fluorphenyldiazoniumtetrafluoroborat.

### Beispiel 2:

60.0 g 4-Fluorphenyldiazoniumtetrafluoroborat werden bei 50°C zu einer Lösung von 57.5 g Methacrylsäuremethylester und 1.65 g (1 Mol%) Palladiumbisbenzylidenacetonkomplex in 200 ml Methanol gegeben. Die Zugabe erfolgte portionsweise. Anschließend wird eine Stunde bei 60°C gerührt. Zur Isolierung des Produktes wird der Katalysator abfiltriert, die Reaktionsmischung im Vakuum eingeengt und nach Verdünnen mit Dichlormethan mit Wasser gewaschen. Das Rohprodukt wird im Vakuum destilliert. Man erhält 49.9 g eines 1.6:1 -Gemisches von 3-(p-Fluorphenyl)methacrylsäuremethylester und 2-(p-Fluorbenzyl)acrylsäuremethylester.
Ausbeute: 90 % 3-(p-Fluorphenyl)methacrylsäuremethylester und 2-(p-Fluorbenzyl)acrylsäuremethylester.
¹H-NMR für 3-(p-Fluorphenyl)methacrylsäure-methylester:
   2,04 (d, J = 1.5 Hz, 3H, CH₃), 3,80 (s, 3H, CO₂CH₃), 6,82-7,40 (m, 4H, Phenyl-H), 7,62 (s, br., 1H, Vinyl-H).
¹H-NMR für 2-(p-Fluorbenzyl)acrylsäure-methylester:
   3,55 (s, br., 2H, Benzyl-H), 3,74 (s, 3H, CO₂CH₃), 5,47, 6,23 (2m, 2H, =CH₂), 6,83-7,38 (m, 4H, Phenyl-H).

### Beispiel 3:

10.0 g 4-Fluorphenyldiazoniumtetrafluoroborat werden bei 50°C zu einer Lösung von 19.3 g Methacrylsäuremethylester und 69 mg (0.25 Mol%) Palladiumbisbenzylidenacetonkomplex in 50 ml Methanol gegeben. Die Zugabe erfolgte portionsweise, nach der Hälfte der Zugabe werden erneut 69 mg (0.25 Mol%) Palladiumkatalysator zugegeben. Anschließend wird eine Stunde bei 60°C gerührt.
Zur Isolierung des Produktes wird der Katalysator abfiltriert, die Reaktionsmischung im Vakuum eingeengt und nach Verdünnen mit Dichlormethan mit Wasser gewaschen. Das Rohprodukt wird chromatographiert.
Man erhält 7.99 g eines 1.5:1-Gemisches von 3-(p-Fluorphenyl)methacrylsäuremethylester und 2-(p-Fluorbenzyl)acrylsäuremethylester.
Ausbeute: 87 % 3-(p-Fluorphenyl)methacrylsäuremethylester und 2-(p-Fluorbenzyl)acrylsäuremethylester.

### Beispiel 4:

10.0 g 4-Fluorphenyldiazoniumtetrafluoroborat werden bei 0°C zu einer Lösung von 19.3 g Methacrylsäuremethylester in 50 ml Methanol gegeben. Die Reaktionsmischung wird auf 50°C erwärmt und portionsweise mit 138 mg (0.25 Mol%) Palladiumbisbenzylidenacetonkomplex versetzt. Anschließend wird eine Stunde bei 60°C gerührt.
Zur Isolierung des Produktes wird der Katalysator abfiltriert, die Reaktionsmischung im Vakuum eingeengt und nach Verdünnen mit Dichlormethan mit Wasser gewaschen. Das Rohprodukt wird chromatographiert.
Man erhält 7.00 g eines 1.5:1-Gemisches von 3-(p-Fluorphenyl)methacrylsäuremethylester und 2-(p-Fluorbenzyl)acrylsäuremethylester.
Ausbeute: 75 % 3-(p-Fluorphenyl)methacrylsäuremethylester und 2-(p-Fluorbenzyl)acrylsäuremethylester.

### Beispiel 5:

60.0 g 4-Fluorphenyldiazoniumtetrafluoroborat werden bei 50°C zu einer Lösung von 57.5 g Methacrylsäuremethylester und 330 mg (0.2 Mol%) Palladiumbisbenzylidenacetonkomplex in 200 ml Methanol gegeben. Die Zugabe erfolgte portionsweise, nach der Hälfte der Zugabe werden erneut 165 mg (0.1 Mol%) Palladiumkatalysator zugegeben. Anschließend wird eine Stunde bei 60°C gerührt.
Zur Isolierung des Produktes wird der Katalysator abfiltriert, die Reaktionsmischung im Vakuum destilliert. Man erhält 48.8 g eines 1.6:1-Gemisches von 3-(p-Fluorphenyl)-methacrylsäuremethylester und 2-(p-Fluorbenzyl)acrylsäuremethylester.
Ausbeute: 88 % 3-(p-Fluorphenyl)methacrylsäuremethylester und 2-(p-Fluorbenzyl)acrylsäuremethylester.

### Beispiel 6:

10.0 g 4-Fluorphenyldiazoniumtetrafluoroborat werden bei 50°C zu einer Lösung von 19.3 g Methacrylsäuremethylester und 0.25 Mol% Palladiumbis-3,3',4,4'-tetramethoxybenzylidenacetonkomplex in 50 ml Methanol gegeben. Die Zugabe erfolgte portionsweise, nach der Hälfte der Zugabe werden erneut 0.1 Mol% Palladiumkatalysator zugegeben. Anschließend wird eine Stunde bei 60°C gerührt.
Zur Isolierung des Produktes wird der Katalysator abfiltriert, die Reaktionsmischung im Vakuum eingeengt und nach Verdünnen mit Dichlormethan mit Wasser gewaschen. Das Rohprodukt wird chromatographiert.
Man erhält 8.54 g eines 1.7:1-Gemisches von 3-(p-Fluorphenyl)-methacrylsäuremethylester und 2-(p-Fluorbenzyl)acrylsäuremethylester.
Ausbeute: 93 % 3-(p-Fluorphenyl)methacrylsäuremethylester und 2-(p-Fluorbenzyl)acrylsäuremethylester.

### Beispiel 7:

10.0 g 4-Fluorphenyldiazoniumtetrafluoroborat werden bei 50°C zu einer Lösung von 19.3 g Methacrylsäuremethylester und 69 mg (0.25 Mol%) Palladiumbisbenzylidenacetonkomplex in 50 ml Ethanol gegeben. Die Zugabe erfolgte portionsweise, nach der Hälfte der Zugabe werden erneut 69 mg (0.25 Mol%) Palladiumkatalysator zugegeben. Anschließend wird eine Stunde bei 60°C gerührt.
Zur Isolierung des Produktes wird der Katalysator abfiltriert, die Reaktionsmischung im Vakuum eingeengt und nach Verdünnen mit Dichlormethan mit Wasser gewaschen. Das Rohprodukt wird chromatographiert.
Ausbeute: 84 % 3-(p-Fluorphenyl)methacrylsäuremethylester und 2-(p-Fluorbenzyl)acrylsäuremethylester.

### Beispiel 8:

50 g 4-Fluoranilin werden in 173 g Tetrafluorborsäure (50 %ig) gelöst und 10 Minuten bei 0 bis 5°C gerührt. Dann addiert man 33,5 g Natriumnitrit in 40 ml Wasser innerhalb von 20 Minuten bei 0 bis 5°C zu. Anschließend fügt man 0,5 g Harnstoff, 100 ml Methanol, 2,58 g Palladiumbisdibenzylidenacetonkomplex und 180 g Methylmethacrylat zur Mischung. Die Reaktionsmischung wird auf 60-70°C erwärmt und bis zur Beendigung der Stickstoffentwicklung bei dieser Temperatur gerührt.
Anschließend werden die Phasen getrennt und die wäßrige Phase mit Essigester extrahiert. Die vereinigten organischen Phasen werden destilliert.
Ausbeute: 82 % 3-(p-Fluorphenyl)methacrylsäuremethylester und 2-(p-Fluorbenzyl)acrylsäuremethylester.

### Beispiel 9:

Eine Mischung von 10,0 g 3-(p-Fluorphenyl)methacrylsäuremethylester und 2-(p-Fluorbenzyl)acrylsäuremethylester werden mit 0,5 g Palladium auf Aktivkohle (5 %ig) in 30 ml Methanol 3 Stunden im Autoklaven bei 50°C gerührt. Nach Abkühlen und filtrieren des Katalysators wird das Produkt durch Chromatographie gereinigt.
Ausbeute: 92 % 3-(4-Fluorphenyl)propionsäuremethylester.

## Patentansprüche

1. Verfahren zur Herstellung von 3-(p-Fluorphenyl)-2-methyl-propionsäure und deren Derivaten der Formel (I), 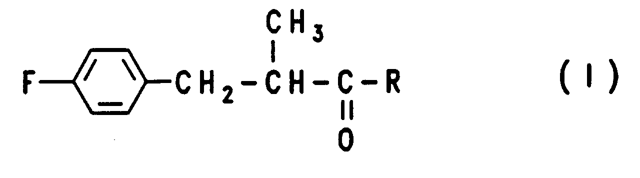 in welcher R eine Hydroxygruppe, eine lineare oder verzweigte (C₁-C₈)-Alkoxygruppe, eine Aryloxygruppe, eine Aminogruppe oder eine (C₁-C₈)-Alkylaminogruppe oder eine (C₁-C₈)-Dialkylaminogruppe darstellt, dadurch gekennzeichnet, daß man
a) p-Fluoranilin in das p-Fluorphenyldiazoniumsalz der Formel (II) überführt 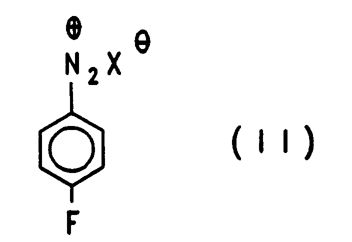 in welcher X das Äquivalent eines Anions einer organischen oder anorganischen Säure mit einem pKa-Wert kleiner 7 darstellt
b) das Diazoniumsalz der Formel (II) mit einem Methacrylsäurederivat der Formel (III) 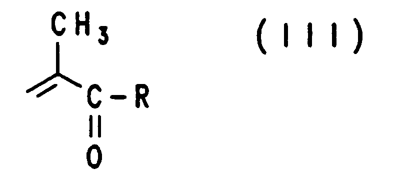 in welcher R die oben angegebene Bedeutung besitzt, in Gegenwart eines Palladiumkatalysators, gegebenenfalls in Gegenwart einer Base und/oder eines Lösungsmittels wobei die Verwendung einer Carbonsäure ausgeschlossen ist, zu den Verbindungen der Formeln (IV) und (V), in welchen R die oben angegebene Bedeutung besitzt, umsetzt 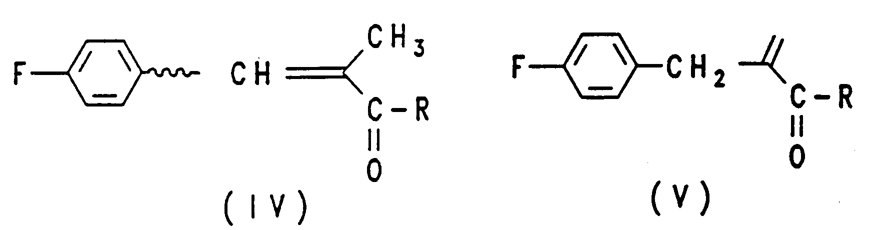 und
c) die erhaltenen Verbindungen der Formeln (IV) und (V) in Gegenwart eines Palladiumkatalysators mit Wasserstoff hydriert.

2. Verfahren nach Anspruch 1, dadurch gekennzeichnet, daß Schritt a) und Schritt b) als Eintopfreaktion durchgeführt werden.

3. Verfahren nach Anspruch 1 oder 2, dadurch gekennzeichnet, daß man die Umsetzung mit dem Methacrylsäurederivat der Formel (III) bei Temperaturen von 0 bis 120°C, insbesondere 20 bis 100°C, bevorzugt 30 bis 80°C durchführt.

4. Verfahren nach mindestens einem der Ansprüche 1 bis 3, dadurch gekennzeichnet, daß man für Schritt b) den Palladiumkatalysator in heterogener Form einsetzt.

5. Verfahren nach Anspruch 4, dadurch gekennzeichnet, daß man den Palladiumkatalysator auf einem Trägermaterial, insbesondere Aktivkohle, Calciumcarbonat, Bariumsulfat, Bimsstein, Tonerde, Kieselgur, Kieselgel, Graphit, Magnesiumoxid und/oder Aluminiumoxid, bevorzugt Aktivkohle anwendet.

6. Verfahren nach Anspruch 4, dadurch gekennzeichnet, daß man Palladiumbisbenzylidenacetonverbindungen in heterogener Form einsetzt.

7. Verfahren nach mindestens einem der Ansprüche 1 bis 6, dadurch gekennzeichnet, daß man in Schritt b) 0.001 bis 1 Mol%, insbesondere 0.01 bis 0.8 Mol% Palladium, bezogen auf das Diazoniumsalz, einsetzt.

8. Verfahren nach mindestens einem der Ansprüche 1 bis 7, dadurch gekennzeichnet, daß man in Schritt b) 1 Mol Diazoniumsalz mit 0.8 bis 20 Mol, insbesondere 1 bis 5 Mol Methacrylsäurederivet umsetzt.

9. Verfahren nach mindestens einem der Ansprüche 1 bis 8, dadurch gekennzeichnet, daß man in Schritt b) als Lösungsmittel Wasser, Methanol, Ethanol oder Gemische davon einsetzt.

## Claims

1. A process for preparing 3-(p-fluorophenyl)-2-methyl-propionic acid and derivatives thereof of the formula (I), 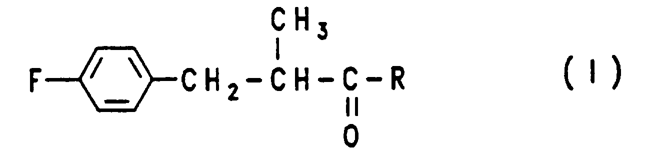 in which R is a hydroxy group, a linear or branched (C₁-C₈)-alkoxy group, an aryloxy group, an amino group or a (C₁-C₈) -alkylamino group or a (C₁-C₈)-dialkylamino group, which process comprises
a) converting p-fluoroaniline into the p-fluorophenyldiasonium salt of the formula (II) 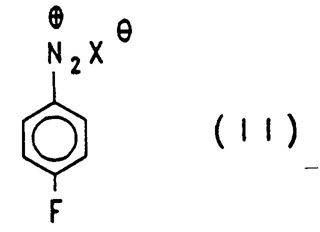 in which X is the equivalent of an anion of an organic or inorganic acid having a pKa value of less than 7,
b) reacting the diazonium salt of the formula (II) with a methacrylic acid derivative of the formula (III) 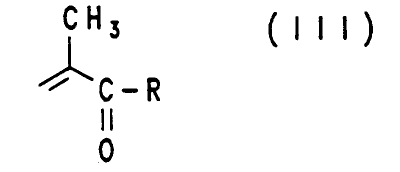 in which R is as defined above in the presence of a palladium catalyst, if desired in the presence of a base and/or a solvent, disclaiming the use of a carboxylic acid, to give the compounds of the formulae (IV) and (V) in which R is as defined above, 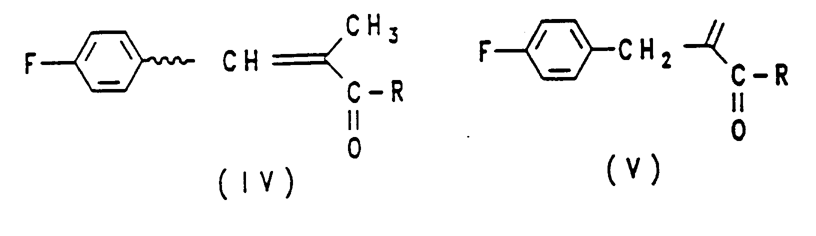 and
c) hydrogenating the resulting compounds of the formulae (IV) and (V) in the presence of a palladium catalyst with hydrogen.

2. The process as claimed in claim 1, wherein step a) and step b) are carried out as a one-pot reaction.

3. The process as claimed in claim 1 or 2, wherein the reaction with the methacrylic acid derivative of the formula (III) is carried out at temperatures of 0 to 120°C, in particular 20 to 100°C, preferably 30 to 80°C.

4. The process as claimed in at least one of claims 1 to 3, wherein for step b) the palladium catalyst is used in heterogeneous form.

5. The process as claimed in claim 4, wherein the palladium catalyst is used on a support, in particular activated carbon, calcium carbonate, barium sulfate, pumice, clay, kieselguhr, silica gel, graphite, magnesium oxide and/or alumina, preferably activated carbon.

6. The process as claimed in claim 4, wherein palladium bis(benzylidene)acetone compounds are used in heterogeneous form.

7. The process as claimed in at least one of claims 1 to 6, wherein in step b) 0.001 to 1 mol%, in particular 0.01 to 0.8 mol%, of palladium, relative to the diazonium salt, is used.

8. The process as claimed in at least one of claims 1 to 7, wherein in step b) 1 mol of diazonium salt is reacted with 0.8 to 20 mol, in particular 1 to 5 mol, of methacrylic acid derivative.

9. The process as claimed in at least one of claims 1 to 8, wherein in step b) the solvent used is water, methanol, ethanol or mixtures thereof.

## Revendications

1. Procédé pour la préparation d'acide 3-(p-fluorophényl)-2-méthyl-propionique et de ses dérivés de formule (I), 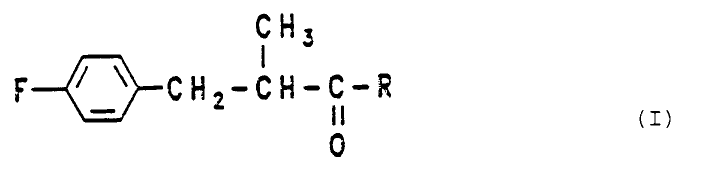 dans laquelle R est un groupe hydroxyle, un groupe alcoxy en C₁-C₈ linéaire ou ramifié, un groupe aryloxy, un groupe amino ou un groupe (alkyl en C₁-C₈) amino ou un groupe di (alkyle en C₁-C₈) amino, caractérisé en ce que
a) on transforme la p-fluoraniline en le sel de diazonium de p-fluorophényle de formule (II) 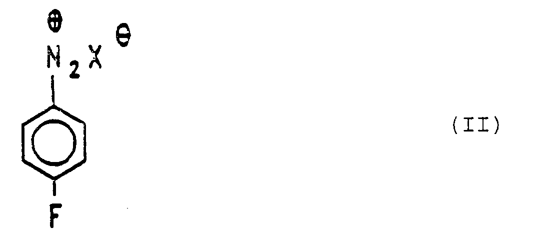 dans laquelle X représente l'équivalent d'un anion d'un acide organique ou inorganique avec une valeur pKa inférieure à 7,
b) le sel de diazonium de formule (II) avec un dérivé d'acide méthacrylique de formule (III) 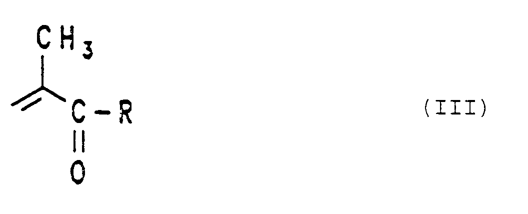 dans laquelle R a la signification donnée ci-dessus, en présence d'un catalyseur au palladium, éventuellement en présence d'une base et/ou d'un solvant, l'utilisation d'un acide carboxylique étant exclue, pour obtenir des composés de formules (IV) et (V), dans lesquelles R a la signification donnée ci-dessus, 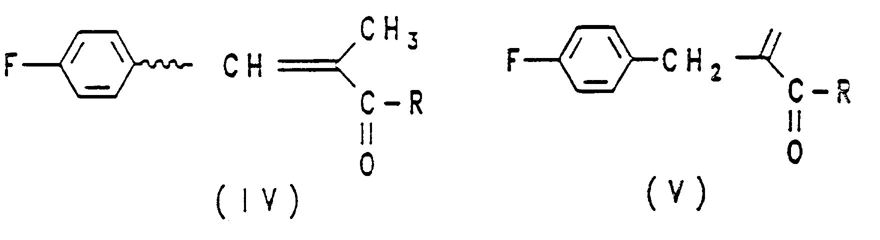
c) on hydrogène les composés obtenus de formules (IV) et (V) en présence d'un catalyseur au palladium par l'hydrogène.

2. Procédé selon la revendication 1, caractérisé en ce que l'on met en oeuvre l'étape a) et l'étape b) comme une réaction à un réacteur.

3. Procédé selon la revendication 1 ou 2, caractérisé en ce que l'on met en oeuvre la réaction avec le dérivé d'acide méthacrylique de formule (III) à une température de 0 à 120 °C, plus particulièrement à une température de 20 à 100 °C, de préférence de 30 à 80 °C.

4. Procédé selon au moins l'une des revendications 1 à 3, caractérisé en ce que l'on utilise dans l'étape b) le catalyseur au palladium sous forme hétérogène.

5. Procédé selon la revendication 4, caractérisé en ce que l'on utilise le catalyseur au palladium sur une matière de support, plus particulièrement le charbon actif, le carbonate de calcium, le sulfate de baryum, la pierre-ponce, l'argile, le kieselgur, le gel de silice, le graphite, l'oxyde de magnésium et/ou l'oxyde d'aluminium, de préférence le charbon actif.

6. Procédé selon la revendication 4, caractérisé en ce que l'on utilise les composés de palladium-bis-benzylidène-acétone sous forme hétérogène.

7. Procédé selon au moins l'une des revendications 1 à 6, caractérisé en ce que l'on utilise dans l'étape b) de 0,001 à 1 % en moles, plus particulièrement de 0,01 à 0,8 % en moles de palladium par rapport au sel de diazonium.

8. Procédé selon au moins l'une des revendications 1 à 7, caractérisé en ce que l'on fait réagir à l'étape b) 1 mole de sel de diazonium avec 0,8 à 20 moles, plus particulièrement de 1 à 5 moles de dérivé d'acide méthacrylique.

9. Procédé selon au moins l'une des revendications 1 à 8, caractérisé en ce que l'on utilise à l'étape b), en tant que solvant, l'eau, le méthanol, l'éthanol, ou leurs mélanges.
